# EUROPEAN PATENT SPECIFICATION

(11) **EP 2 109 439 B1**
(45) Date of publication and mention of the grant of the patent: **13.04.2011**
(21) Application number: 08837715.5
(22) Date of filing: 07.10.2008
(51) Int. Cl.: A61K 8/368, A61K 8/41, A61K 8/43

(54) **OPHTHALMIC COMPOSITIONS WITH A DISUCCINATE**
OPHTHALMISCHE ZUSAMMENSETZUNGEN MIT EINEM DISUCCINAT
COMPOSITIONS OPHTALMIQUE AVEC UN DISUCCINATE

(30) Priority: 08.10.2007 US 978171 P; 07.10.2008 US 246522
(43) Date of publication of application: 21.10.2009
(73) Proprietor: Bausch & Lomb Incorporated, Rochester, NY 14604-2701 (US)
(72) Inventor: MACLEOD, Steven, K., Henrietta NY 14467 (US)
(74) Representative: Vossius & Partner
(86) International application number: PCT/US2008/079032
(87) International publication number: WO 2009/048860

(56) References cited:
- WO-A-02/49615
- WO-A-98/32421
- WO-A-2008/049042
- US-A- 5 494 937

## Description

The present invention relates to ophthalmic compositions with a disuccinate. The invention is also directed to the use of the ophthalmic compositions as a component in disinfecting compositions, particularly compositions used to clean and disinfect contact lenses.

### Background of the Invention

Aqueous ophthalmic solutions are typically applied to eyes in the form of drops, or used to treat contact lenses that are subsequently placed in the eye. The primary functions of these solutions are to provide a moisturizing effect for eyes, or to clean, disinfect or wet contact lenses. Given these primary functions, ophthalmic solutions will typically include one or more antimicrobial components, one or more surfactants and one or more chelating agents.

There remains an interest and need for improved contact lens care solutions that offer a greater comfort level to the patient without sacrificing antimicrobial efficacy and cleaning ability.

### Summary of the Invention

The invention is directed to ophthalmic compositions comprising a disuccinate of formula I or a corresponding salt thereof; 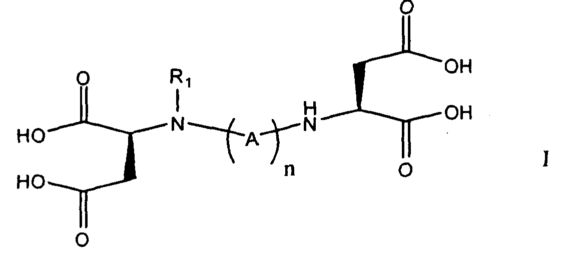 wherein R₁ is selected from hydrogen, alkyl or -C(O)alkyl, the alkyl having one to twelve carbons and optionally one or more oxygen atoms, A is a methylene group or an oxyalkylene group, and n is from 2 to 8. The ophthalmic compositions also include an antimicrobial component and a tonicity agent, wherein the tonicity agent provides for an osmolality of the composition from 200 mOs/kg to 420 mOsm/kg.

### Brief Description of the Figure

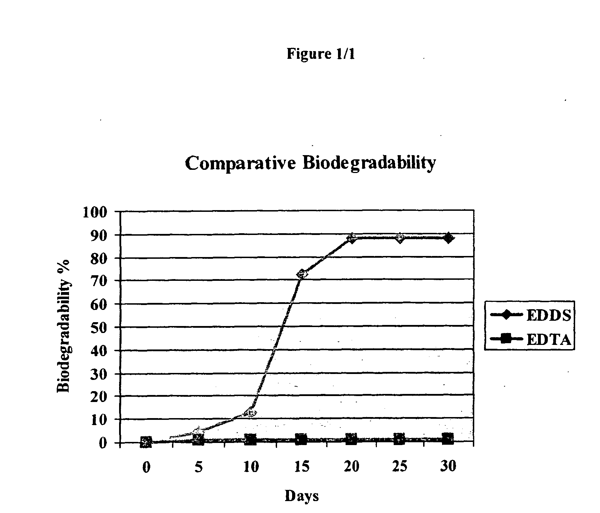

Figure 1 is a graphical representation of the biodegradability of EDDS vs. EDTA.

### Detailed Description of the Invention

Three of the four leading contact lens care solutions sold in the U.S. contain disodium ethylenediamine tetraacetic acid (Na₂EDTA), however, Na₂EDTA is not biodegradable. Applicants have sought biodegradable alternatives and discovered that disuccinate agents can be used in contact lens care solutions with little or no effect on the biocidal efficacy or the cleaning ability of the solutions.

Accordingly, the invention is directed to ophthalmic compositions comprising a disuccinate of formula I or a corresponding salt thereof; 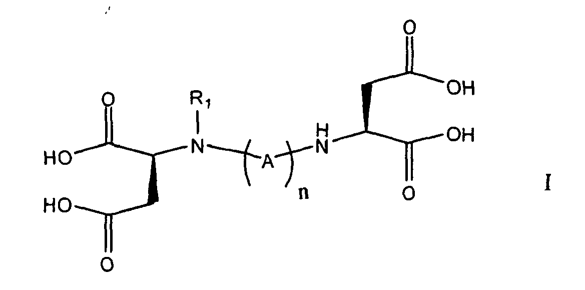 wherein R₁ is selected from hydrogen, alkyl or -C(O)alkyl, the alkyl having one to twelve carbons and optionally one or more oxygen atoms, A is a methylene group or an oxyalkylene group, and n is from 2 to 8. The ophthalmic compositions also include an antimicrobial component and a tonicity agent, wherein the tonicity agent provides for an osmolality of the composition from 200 mOs/kg to 420 mOsm/kg.

In one embodiment, the disuccinate present in the composition is S,S-ethylenediamine disuccinate (S,S-EDDS) or a corresponding salt thereof. One commercial source of S,S-EDDS is represented by Octaquest® E30, which is commercially available from Octel. The chemical structure of the trisodium salt of S,S-EDDS is shown below. 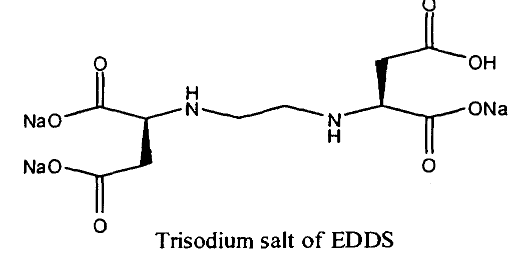 Typically, the disuccinate is added with the other aqueous components of an ophthalmic composition as its corresponding salt. The salts can include the alkali metals of Group IA such as sodium and potassium. The salts can also include the alkaline earth metals such as calcium or magnesium. The zinc or silver salt of the disuccinate can also be used in the ophthalmic compositions.

The disuccinate compounds of formula I can be prepared by the reaction of a suitable bridging compound, e.g., any alkyl with terminal leaving groups such as 1,2-dichloroethane, with aspartic acid under basic reaction conditions. The disuccinate compounds of formula I with the acyl group, i.e., -C(O)alkyl, are easily prepared from a disuccinate diamine and a suitable acylchloride. For example, one particular disuccinate of formula 1 includes A as a methylene with n is 2, 3 or 4, and -C(O)alkyl has eight to twelve carbons.

### The Use of the Dissucinate of Formula I in Contact Lens Care Compositions

In one embodiment, the disuccinate is present as one component of an ophthalmic lens care solution that is used to clean, disinfect or package contact lenses. In this case, the lens care solution will include one or more antimicrobial components along with other solution components that provide additional properties required of such solutions.

The antimicrobial component is present in an amount from 0.05 ppm to 50 ppm or from 0.1 ppm to 10 ppm. It is preferred, however, that the amount of antimicrobial component that is used is effective in disinfecting contact lenses contacted with the compositions, while at the same time not contributing to patient discomfort. Typically, an amount of the antimicrobial component is used to reduce the microbial burden or load on the contact lens by one log order in four hours. Alternatively, an effective amount of the antimicrobial component reduces the microbial load by one log order in one hour. The reductions are based upon similarly prepared lens solutions absent the cationic antimicrobial component.

One class of antimicrobial components are referred to as cationic antimicrobial components. Suitable cationic antimicrobial components include, but are not limited to, quaternary ammonium salts used in ophthalmic applications such as α-[4-tris(2-hydroxyethyl) ammonium chloride-2-butenyl]poly[1-dimethylammonium chloride-2-butenyl]-ω-tris(2-hydroxyethyl)ammonium chloride (CAS# 68518-54-7, available as Polyquaternium-1® from Stepan Corporation), benzalkonium halides, and biguanides such as salts of alexidine, alexidine-free base, salts of chlorhexidine, polymeric biguanides such as poly(hexamethylenebiguanide) (PHMB), antimicrobial polypeptides and mixtures thereof. The term "cationic" when referring to an antimicrobial component refers to the predominant form of the antimicrobial component at neutral pH having a positive charge and a counteranion.

In one embodiment, one cationic antimicrobial component present in a lens care solution is a polymeric biguanide, which is present from 0.01 ppm to 3 ppm. In another embodiment, the lens care solution will also include α-[4-tris(2-hydroxyethyl)ammonium chloride-2-butenyl]poly[1-dimethylammonium chloride-2-butenyl]-ω-tris(2-hydroxyethyl)ammonium chloride, which is present from 1 ppm to 10 ppm. A defined mixture of the two cationic antimicrobial components in a solution can provide additional advantages. For example, a particular lens care solution can include from 0.3 ppm to 0.8 ppm of a polymeric biguanide, and 3 ppm to 8 ppm α-[4-tris(2-hydroxyethyl)ammonium chloride-2-butenyl]poly[1-dimethylammonium chloride-2-butenyl]-ω-tris(2-hydroxyethyl)ammonium chloride.

The lens care solutions can also include a phosphonic acid, or its physiologically compatible salt, that is represented by the following formula: 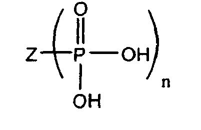 wherein Z is a connecting radical equal, n is an integer from 1 to 4, or 1, 2 or 3, and preferably containing 1 to 12 carbon atoms, more preferably 3 to 10 carbon atoms. The Z radical comprises substituted or unsubstituted saturated hydrocarbon radicals or amine-containing radicals, which amine-containing radicals are saturated hydrocarbon radicals in which the carbon atoms are interrupted with at least one nitrogen atom such as 1, 2 or 3 nitrogen atoms that forms a secondary or tertiary amine.

Accordingly, suitable Z radicals include substituted or unsubstituted alkylidene, substituted or unsubstituted alkylene, amino tri(alkylene) having at least n+1 carbon atoms, amino di(alkylene) having at least n+1 carbon atoms, alkylenediaminetetra(alkylene) or a dialkylenetriamine penta(alkylene) radical. In each case, the alkylene group in parenthesis is connected to a phosphonic acid group. Preferably, all alkylene groups independently have 1 to 4 carbon atoms.

Exemplary compounds in which the Z group is an amino tri(alkylene) radical includes amino tri(ethylidene phosphonic acid), amino tri(isopropylidene phosphonic acid), amino di(methylene phosphonic acid) mono(isopropylidene phosphonic acid), and amino mono(methylene phosphonic acid) di(ethylidene phosphonic acid). Exemplary compounds in which the Z group is a substituted or unsubstituted alkylidene radical includes methylene diphosphonic acid, ethylidine diphosphonic acid, 1-hydroxy propylidene diphosphonic acid. Exemplary compounds in which the Z group is an alkylenediaminetetra(alkylene) or a dialkylenetriamine penta(alkylene) radical include hexamethylenediaminetetra(methylene phosphonic acid) and diethylenetriaminepenta(methylenephosphonic acid).

In one embodiment, the phosphonic acid, or its physiologically compatible salt, is represented by the following formula: 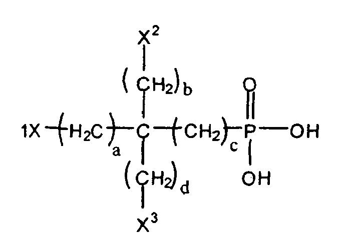 wherein each of a, b, c, and d are independently selected from integers from 0 to 4, preferably 0 or 1; X¹ is a phosphonic acid group (i.e., P(OH)₂O), hydroxy, amine or hydrogen; and X² and X³ are independently selected from the group consisting of halogen, hydroxy, amine, carboxy, alkylcarbonyl, alkoxycarbonyl, or substituted or unsubstituted phenyl, and methyl. Exemplary substituents on the phenyl are halogen, hydroxy, amine, carboxy and/or alkyl groups. A particularly preferred species is that wherein a, b, c, and d in are zero, specifically the tetrasodium salt of 1-hydroxyethylidene-1,1-diphosphonic acid, also referred to as tetrasodium etidronate, commercially available from Monsanto Company as DeQuest® 2016 diphosphonic acid sodium salt or phosphonate.

A stable tear film can be critical to prevent pathogenic microorganism invasion.
Microorganism invasion can be facilitated by an epithelial defect or an unstable tear film. A stable preocular tear film depends on many factors, including the correct quantity and quality of various components of the tears and the integrity of the corneal epithelium. Environmental pollution, smoking and frequent use of eye drops can cause denaturization of tear proteins such as lysozyme and lactoferrin. It has been proposed that denatured tear proteins can cause destabilization of tear film, staining, loss of tight junction, and dry eye.

Accordingly, the contact lens care solutions can include at least one epithelium cell stabilizer selected from the group consisting of diglycine, glycine, triglycine, tetraglycine and pentaglycine. The epithelium cell stabilizer is generally present in the solution at a concentration of from 0.00 1 % to a 10% (w/v), for instance 0.1% to 5% (w/v) or 0.1% to 2 % (w/v).

The lens care solutions can include dexpanthenol, which is an alcohol of pantothenic acid, also called Provitamin B5, D-pantothenyl alcohol or D-panthenol. It has been stated that dexpanthenol may play a role in stabilizing the lachrymal film at the eye surface following placement of a contact lens on the eye. Dexpanthenol is preferably present in the solution in an amount from 0.2% to 10% (w/v), from 0.5% to 5% (w/v), or from 1% to 3% (w/v).

The contact lens care solutions can also include sorbitol, which is a hexavalent sugar alcohol. Typically, dexpanthenol is used in combination with sorbitol. Sorbitol is present in the lens care compositions in an amount from 0.4% to 10% (w/v), from 0.8% to 6% (w/v) or from 1% to 3% (w/v).

The lens care solutions can also include one or more neutral or basic amino acids. The neutral amino acids include: the alkyl-group-containing amino acids such as alanine, isoleucine, valine, leucine and proline; hydroxyl-group-containing amino acids such as serine, threonine and 4-hydroxyproline; thio-group-containing amino acids such as cysteine, methionine and asparagine. Examples of the basic amino acid include lysine, histidine and arginine. The one or more neutral or basic amino acids are present in the compositions at a total concentration of from 0.1% to 5% (w/v).

The lens care solutions can also include glycolic acid, asparatic acid or any mixture of the two at a total concentration of from 0.00 1 % to 4% (w/v) or from 0.0 1 % to 2.0% (w/v).

In addition, the combined use of one or more amino acids and glycolic acid and/or asparatic acid can lead to a reduction in the change of the size of the contact lens due to swelling and shrinkage following placement of the lens on the eye. The stated combination provides a higher degree of compatibility with the contact lens compared to the absence of one of the two components in the solution. It is believed that one or more of the amino acids can cause the lens to swell, and that the glycolic acid and/or asparatic acid can cause the contact lens to shrink. If used in combination, however, a mutual counteraction of the two observed affects is believed to exist.

The lens care solutions can also include glycolic acid, asparatic acid or any mixture of the two, in combination with 2-amino-2-methyl-1,3-propanediol or a salt thereof. In some cases, solutions that contain a mixture of two of the three, or all three, compounds minimize the change of the lens size following placement of the contact lens in the eye. The 2-amino-2-methyl-1, 3-propanediol (AMPD) or the salt thereof is added to the solutions in an amount to satisfy a predetermined molar ratio of glycolic acid, asparatic acid or any mixture of the two and AMPD. The molar ratio of the two components glycolic acid and/or asparatic acid to AMPD is 1:20 to 1.3:1. The glycolic acid, asparatic acid or any mixture of the two is present in the compositions at a concentration of 0.01% to 5% (w/v) or at a concentration of 0.05% to 1% (w/v).

The amount of AMPD present in the solutions can be determined according to the amount of glycolic acid and/or asparatic acid in the solution. As stated, AMPD is present in an amount to provide a molar ratio of glycolic acid and/or asparatic acid to AMPD to be from 1:20 to 1.3:1, from 1:15 to 1.2:1 or from 1:14 to 1:1. If the amount of AMPD exceeds 20 mols per 1 mol of glycolic acid and/or asparatic, adsorption of the cationic antimicrobial component on the contact lens is likely to occur. If the amount of AMPD is less than I mol per 1.3 mols of glycolic acid and/or asparatic acid, a reduction in antimicrobial efficacy of the solution is observed.

The contact lens care solutions will very likely include a buffer system. By the terms "buffer" or "buffer system" is meant a compound that, usually in combination with at least one other compound, provides a buffering system in solution that exhibits buffering capacity, that is, the capacity to neutralize, within limits, either acids or bases (alkali) with relatively little or no change in the original pH. Generally, the buffering components are present from 0.05% to 2.5% (w/v) or from 0.1% to 1.5% (w/v).

The term "buffering capacity" is defined to mean the millimoles (mM) of strong acid or base (or respectively, hydrogen or hydroxide ions) required to change the pH by one unit when added to one liter (a standard unit) of the buffer solution. The buffer capacity will depend on the type and concentration of the buffer components. The buffer capacity is measured from a starting pH of 6 to 8, preferably from 7.4 to 8.4.

Borate buffers include, for example, boric acid and its salts, for example, sodium borate or potassium borate. Borate buffers also include compounds such as potassium tetraborate or potassium metaborate that produce borate acid or its salt in solutions. Borate buffers are known for enhancing the efficacy of certain polymeric biguanides. For example, U.S. Pat. No. 4,758,595 to Ogunbiyi et al. describes that a contact-lens solution containing a polyaminopropyl biguanide (PAPB), also known as PHMB, can exhibit enhanced efficacy if combined with a borate buffer.

A phosphate buffer system preferably includes one or more monobasic phosphates, dibasic phosphates and the like. Particularly useful phosphate buffers are those selected from phosphate salts of alkali and/or alkaline earth metals. Examples of suitable phosphate buffers include one or more of sodium dibasic phosphate (Na₂HPO₄), sodium monobasic phosphate (NaH₂PO₄) and potassium monobasic phosphate (KH₂PO₄). The phosphate buffer components frequently are used in amounts from 0.01% or to 0.5% (w/v), calculated as phosphate ion.

Other known buffer compounds can optionally be added to the lens care compositions, for example, citrates, citric acid, sodium bicarbonate, TRIS, and the like. Other ingredients in the solution, while having other functions, may also affect the buffer capacity.

A preferred buffer system is based upon boric acid/borate, a mono and/or dibasic phosphate salt/phosphoric acid or a combined boric/phosphate buffer system. For example a combined boric/phosphate buffer system can be formulated from a mixture of boric acid/sodium borate and a monobasic/dibasic phosphate. In a combined boric/phosphate buffer system, the phosphate buffer is used (in total) at a concentration of 0.004 to 0.2 M (Molar), preferably 0.04 to 0.1 M. The borate buffer (in total) is used at a concentration of 0.02 to 0.8 M, preferably 0.07 to 0.2 M.

The lens care solutions will very likely comprise effective amounts of one or more known lens care formulation components such as a detergent or surfactant component, a viscosity inducing or thickening component, a chelating or sequestering component, or a tonicity component. The additional component or components can be selected from materials which are known to be useful in contact lens care solutions and are included in amounts effective to provide the desired effect or benefit.

Suitable surfactants can be either amphoteric, cationic, anionic, or nonionic, and are typically present (individually or in combination) in amounts up to 15%, or up to 5% (w/v). One preferred surfactant class are the amphoteric or nonionic surfactants. The surfactant should be soluble in the lens care solution and non-irritating to eye tissues. Many nonionic surfactants comprise one or more chains or polymeric components having oxyalkylene (--O-R--) repeats units wherein R has 2 to 6 carbon atoms. Preferred non-ionic surfactants comprise block polymers of two or more different kinds of oxyalkylene repeat units, which ratio of different repeat units determines the HLB of the surfactant. Satisfactory non-ionic surfactants include polyethylene glycol esters of fatty acids, e.g. coconut, polysorbate, polyoxyethylene or polyoxypropylene ethers of higher alkanes (C₁₂-C₁₈). Examples of the this class include polysorbate 20 (available under the trademark Tween® 20), polyoxyethylene (23) lauryl ether (Brij® 35), polyoxyethyene (40) stearate (Myrj®52), polyoxyethylene (25) propylene glycol stearate (Atlas® G 2612). Still other preferred surfactants include tyloxapol, betaine-type surfactants, polysulfates, polyethylene glycol, alkyl esters and any mixture thereof.

A particular non-ionic surfactant consisting of a poly(oxypropylene)-poly(oxyethylene) adduct of ethylene diamine having a molecular weight from about 7,500 to about 27,000 wherein at least 40 weight percent of said adduct is poly(oxyethylene) has been found to be particularly advantageous for use in cleaning and conditioning both soft and hard contact lenses when used in amounts from about 0.01 to about 15 weight percent. The CTFA Cosmetic Ingredient Dictionary's adopted name for this group of surfactants is poloxamine. Such surfactants are available from BASF Wyandotte Corp., Wyandotte, Mich., under Tetronic®.

An analogous of series of surfactants, for use in the lens care compositions, is the poloxamer series which is a poly(oxyethylene) poly(oxypropylene) block polymers available under Pluronic® (commercially available form BASF). In accordance with one embodiment of a lens care composition the poly(oxyethylene)-poly(oxypropylene) block copolymers will have molecular weights from 2500 to 13,000 daltons or from 6000 to about 12,000 daltons. Specific examples of surfactants which are satisfactory include: poloxamer 108, poloxamer 188, poloxamer 237, poloxamer 238, poloxamer 288 and poloxamer 407. Particularly good results are obtained with poloxamer 237.

Amphoteric surfactants suitable for use in an ophthalmic compositions can also be present in the compositions. These include amphoteric surfactants commercially available under the trade name "Miranol." Another useful class of amphoteric surfactants is exemplified by cocoamidopropyl betaine, commercially available from various sources. Still another class of compounds include the sulphobetaine compounds described in U.S. Patent No. 5,765,579, and in particular, N-decyl-N,N-dimethyl-3-ammonio-1-propane sulfate, available as Zwittergent 3-10 from Calbiochem Co.

The foregoing surfactants will generally be present in a total amount from 0.01% to 5% (w/v), from 0.1% to 5% (w/v), or from 0.1% to 1.5% (w/v). Often the amount of surfactant is from 0.005% or 0.01%, to 0.1% or 0.5% or 0.8% (w/v).

The lens care solutions can also include a viscosity enhancing component. The viscosity inducing components should be compatible with the other components and are preferably nonionic. Such viscosity inducing components are effective to enhance and/or prolong the cleaning and wetting activity of the surfactant component and/or condition the lens surface rendering it more hydrophilic (less lipophilic) and/or to act as a demulcent on the eye. Increasing the solution viscosity provides a film on the lens which may facilitate comfortable wearing of the contact lens. The viscosity inducing component can also function to cushion the impact on the eye surface during placement of the lens and serves also to alleviate eye irritation.

Suitable viscosity inducing components include, but are not limited to, water soluble natural gums, cellulose-derived polymers and the like. Useful natural gums and their derivatives include guar gum, hydroxylpropyl guar gum, gum tragacanth and the like. Useful cellulose-derived viscosity inducing components include cellulose-derived polymers, such as hydroxypropyl cellulose, hydroxypropylmethyl cellulose, carboxymethyl cellulose, methyl cellulose, hydroxyethyl cellulose and the like. A very useful viscosity inducing component is hydroxypropylmethyl cellulose (HPMC).

The viscosity inducing component is used in an amount effective to increase the viscosity of the solution, preferably to a viscosity in the range of about 1.5 to about 30, or even as high as about 750, cps at 25°C, as determined by USP test method No. 911 (USP 23, 1995).

The lens care solutions will typically have an osmolality in the range of at least about 200 mOsmol/kg for example, about 300 or about 350 to about 400 mOsmol/kg. The lens care solutions are substantially isotonic or hypertonic (for example, slightly hypertonic) and are ophthalmically acceptable.

The lens care solutions will typically include an effective amount of a tonicity adjusting component. Among the suitable tonicity adjusting components that can be used are those conventionally used in contact lens care products such as various inorganic salts. Sodium chloride and/or potassium chloride and the like are very useful tonicity components. The amount of tonicity adjusting component is effective to provide the desired degree of tonicity to the solution.

The lens care solutions can also include propylene glycol or glycerin, which with selected formulations has been shown to increase the antimicrobial properties of the solution. This increase in antimicrobial activity allows for a reduction in the amount of antimicrobial component in the solution. The propylene glycol or glycerin is present in the solution from 0.1% to 2% (w/v).

As described, the ophthalmic compositions containing disuccinate can be used as a disinfecting/cleaning contact lens solution. In general, such a method would include contacting or soaking the lenses with the solution for a period of time, typically for a minimum of one to four hours. Although such contacting may be accomplished by simply soaking a lens in the ophthalmic composition, greater preserving, disinfecting and/or cleaning may possibly be achieved if a few drops of the solution are initially placed on each side of the lens, and the lens is rubbed for a period of time, for example, approximately 20 seconds. The lens can then be subsequently immersed within several milliliters of the solution. Preferably, the lens is permitted to soak in the solution for at least four hours. Furthermore, the lens is preferably rinsed with fresh composition after any rubbing step and again after being immersed within the solution. The lenses are removed from the solution, rinsed with the same or a different solution, for example, a preserved isotonic saline solution, and repositioned on the eye.

The contact lens solutions containing the disuccinates can be used with many different types of contact lenses including: (1) hard lenses formed from materials prepared by polymerization of acrylic esters, such as poly(methyl methacrylate) (PMMA), (2) rigid gas permeable (RGP) lenses formed from silicone acrylates and fluorosilicone methacrylates, (3) soft, hydrogel lenses, and (4) non-hydrogel elastomer lenses.

As an example, soft hydrogel contact lenses are made of a hydrogel polymeric material, a hydrogel being defined as a crosslinked polymeric system containing water in an equilibrium state. In general, hydrogels exhibit excellent biocompatibility properties, i.e., the property of being biologically or biochemically compatible by not producing a toxic, injurious or immunological response in a living tissue. Representative conventional hydrogel contact lens materials are made by polymerizing a monomer mixture comprising at least one hydrophilic monomer, such as (meth)acrylic acid, 2-hydroxyethyl methacrylate (HEMA), glyceryl methacrylate, N,N-dimethacrylamide, and N-vinylpyrrolidone (NVP). In the case of silicone hydrogels, the monomer mixture from which the copolymer is prepared further includes a silicone-containing monomer, in addition to the hydrophilic monomer. Generally, the monomer mixture will also include a crosslink monomer such as ethylene glycol dimethacrylate, tetraethylene glycol dimethacrylate, and methacryloxyethyl vinylcarbonate. Alternatively, either the silicone-containing monomer or the hydrophilic monomer may function as a crosslink agent.

The contact lens solutions containing the disuccinates can also be formulated for use as a preservative solution or packaging solution for contact lenses. One of ordinary skill in the art would know how to adjust the solution formulation for each of these respective applications. The lens care solutions in combination with its container or bottle and packaging, including instructions for use in accordance with a specified regimen, provides an improved kit, package, or system for the care of contact lenses.

One exemplary ophthalmic composition is formulated as a contact lens disinfecting solution prepared with the components and amounts of each listed in Table 1.

**Table 1.**

| **Component** | **Minimum** **Amount (wt.%)** | **Maximum** **Amount (wt.%)** | **Preferred** **Amount (wt.%)** |
|---|---|---|---|
| boric acid | 0.10 | 1.0 | 0.64 |
| Sodium borate | 0.01 | 0.20 | 0.09 |
| Sodium chloride | 0.20 | 0.80 | 0.49 |
| EDDS | 0.01 | 0.20 | 0.11 |
| Dequest® | 0 | 0.10 | 0.03 |
| Tetronic® 1107 | 0.05 | 2.0 | 1.00 |
| PHMB | 0.1 | 2ppm | 1 ppm |

Another contact lens solution according to the present invention includes the following ingredients listed in Table 2.

**Table 2.**

| **Component** | **Minimum** **Amount (wt.%)** | **Maximum** **Amount (wt.%)** | **Preferred** **Amount (wt.%)** |
|---|---|---|---|
| Sodium borate | 0.1 | 0.8 | 0.65 |
| Tetronic® 1304 | 0.1 | 1.0 | 0.05 |
| Sodium citrate | 0.1 | 0.6 | 0.45 |
| Sodium chloride | 0.05 | 0.8 | 0.10 |
| boric acid | 0.1 | 1.0 | 0.60 |
| EDDS | 0.01 | 0.20 | 0.05 |
| PHMB | 0.3 ppm | 1.0 ppm | 0.5 ppm |
| Polyquaternium-1 | 1 ppm | 15 ppm | 8 ppm |

Another contact lens solution according to the present invention includes the following ingredients listed in Table 3.

**Table 3.**

| **Component** | **Minimum** **Amount (wt.%)** | **Maximum** **Amount (wt.%)** | **Preferred** **Amount (wt.%)** |
|---|---|---|---|
| Sodium citrate | 0.1 | 0.8 | 0.6 |
| Sodium chloride | 0.05 | 0.8 | 0.1 |
| Sodium borate | 0.10 | 1.0 | 0.60 |
| propylene glycol | 0.2 | 2.0 | 1.00 |
| Tetronic® 1304 | 0.05 | 0.5 | 0.10 |
| EDDS | 0.05 | 0.5 | 0.20 |
| Polyquarternium-1 | 1 ppm | 15 ppm | 10 ppm |

Another contact lens solution according to the present invention includes the following ingredients listed in Table 4.

**Table 4.**

| **Component** | **Minimum** **Amount (wt.%)** | **Maximum** **Amount (wt.%)** | **Preferred** **Amount (wt.%)** |
|---|---|---|---|
| Sorbitol | 0.2 | 2.5 | 1.88 |
| tromethamine | 0.05 | 1.0 | 0.33 |
| Pluronic® F127 | 0.05 | 1.0 | 0.10 |
| sodium phosphate, dihydrogen | 0.10 | 0.8 | 0.46 |
| dexpanthenol | 0.5 | 2.50 | 2.00 |
| EDDS | 0.01 | 0.5 | 0.2 |
| PHMB | 0.5ppm | 2ppm | 1 ppm |

Another contact lens solution according to the present invention includes the following ingredients listed in Table 5.

**Table 5.**

| **Component** | **Minimum** **Amount (wt.%)** | **Maximum** **Amount (wt.%)** | **Preferred** **Amount (wt.%)** |
|---|---|---|---|
| NaCl/KCl | 0.2 | 2.5 | 0.70 |
| propylene glycol | 0.1 | 1.0 | 0.50 |
| poloxamer 237 | 0.01 | 0.20 | 0.05 |
| phosphate monobasic | 0.05 | 0.40 | 0.10 |
| phosphate dibasic | 0.05 | 0.4 | 0.12 |
| HPMC | 0.05 | 0.4 | 0.15 |
| EDDS | 0.05 | 0.5 | 0.2 |
| PHMB | 0.5ppm | 2ppm | 1.1 ppm |

The ophthalmic compositions can also be formulated as a contact lens rewetting eye drop solution. By way of example, the rewetting drops may be formulated according to any one of the foregoing formulations of Tables 1 to 4 above. Alternatively, the formulations may be modified by increasing the amount of surfactant; by reducing the amount of antimicrobial agent to a preservative amount and/or by adding a humectant and/or demulcent.

The ophthalmic compositions can be used as a preservative in formulations for treating patients with dry eye. In such a method, the ophthalmic composition is administered to the patient's eye, eye lid or to the skin surrounding the patient's eye. The compositions can be administered to the eyes irrespective of whether contact lenses are present in the eyes of the patient. For example, many people suffer from temporary or chronic eye conditions in which the eye's tear system fails to provide adequate tear volume or tear film stability necessary to remove irritating environmental contaminants such as dust, pollen, or the like.

The ophthalmic compositions can also be used as a preservative in pharmaceutical compositions such as nasal sprays, ear and eye drops, suppositories, and prescription and over-the-counter formulations containing a pharmaceutical active that are used or administered over time such as a cream, ointment, gel or solution.

In many instances, the ophthalmic compositions will include one or more active pharmaceutical agents. Generally, the active pharmaceutical agent is in one or more classes of ocular pharmaceuticals including, but not limited to anti-inflammatory agents, antibiotics, immunosuppressive agents, antiviral agents, antifungal agents, anesthetics and pain killers, anticancer agents, anti-glaucoma agents, peptide and proteins, anti-allergy agents.

In one embodiment, the active pharmaceutical agent is an anti-inflammatory agent such as a glucocorticosteroid including, but not limited to, alclometasone, algestone, amcinonide, beclomethasone, flucloronide, hydrocortisone, loteprednol etabonate, difluprednate, cortisone and combinations thereof, or a non-steroidal anti-inflammatory agent including, but not limited to, enfenamic acid, aceclofenac, bumadizon, clidanac, alminoprofen, pyrazolones, salicyclic acid, fepradinol, ampiroxicam, and combinations thereof.

In another embodiment, the active pharmaceutical agent is an antibiotic including, but not limited to, doxorubicin, apramycin, biapenem, cefaclor, ceftezole, amdinocillin, clindamycin, carbomycin, clomocycline, cinoxacin, ciprofloxacin, sulfadiazine, and combinations thereof

In still another embodiment, the active pharmaceutical agent is an immunosuppressive agent including, but not limited to, cyclosporin A, gusperimus, fluocinolone, triaminolone, carmofur, azathioprine and combinations thereof.

In still another embodiment, the active pharmaceutical agent is an antiviral agent including, but not limited to, trisodium phosphomonoformate, trifluorothymidine, acyclovir, ganciclovir, and combinations thereof.

In still another embodiment, the active pharmaceutical agent is an antifungal agent including, but not limited to, amphotericin, neomycin, bifonazole, lanoconazole, chlorphenesin, zinc propionate and siccanin.

In still another embodiment, the active pharmaceutical agent is an antiglaucoma agent including, but not limited to, timolol, betaxolol, atenalol, acetylcholine chloride, carbachol, pilocarpine hydrochloride, and combinations thereof.

In still another embodiment, the active pharmaceutical agent is an anti-allergy agent including, but not limited to, phenylephrine hydrochloride, naphazoline hydrochloride, tetrahydrozoline hydrochloride, oxymetazoline hydrochloride, sodium cromoglycate and epinephrine.

## Claims

1. An ophthalmic composition comprising:
a disuccinate of formula I or a corresponding salt thereof: 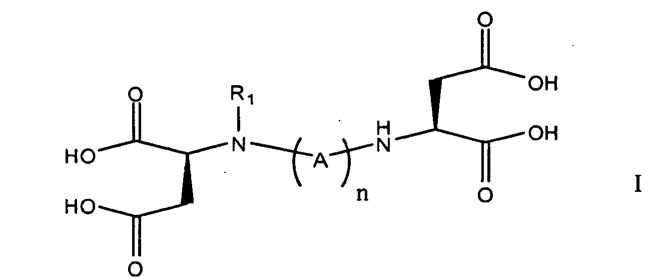 wherein R₁ is selected from hydrogen, alkyl or -C(O)alkyl, the alkyl having one to twelve carbons and optionally having one or more oxygen atoms,
A is a methylene group or an oxyalkylene group, and n is from 2 to 10;
an antimicrobial component; and
a tonicity agent, wherein the tonicity agent provides for an osmolality of the composition from 200 mOs/kg to 420 mOsm/kg.

2. The composition of claim 1 wherein the disuccinate is S,S-ethylenediamine disuccinate. -

3. The composition of claim 1 wherein A is methylene and n is 2, 3 or 4 and the alkyl R₁ has eight to twelve carbons.

4. The composition of claims 1 or 3 wherein the antimicrobial component is a cationic antimicrobial component selected from the group consisting of α-[4-tris(2-hydroxyethyl)ammonium chloride-2-butenyl]poly[1-dimethyl ammonium chloride-2-butenyl]-ω-tris(2-hydroxyethyl)ammonium chloride, benzalkonium halides, alexidine and salts thereof, salts of chlorhexidine, hexamethylene biguanides and salts thereof and their polymers, and mixtures thereof.

5. The composition of claims 1 to 3 wherein the antimicrobial component is selected from the group consisting of poly(hexamethylene biguanide), which is present from 0.01 ppm to 3 ppm, α-[4-tris(2-hydroxyethyl)ammonium chloride-2-butenyl]poly[1-dimethylammonium chloride-2-butenyl]-ω-tris(2-hydroxyethyl) ammonium chloride, which is present from 1 ppm to 10 ppm, and any mixture thereof.

6. The composition of claims 1 to 5 further comprising dexpanthenol, sorbitol, aspartic acid, glycolic acid, 2-amino-2-methyl-1,3-propanediol or any mixture thereof.

7. The composition of claims 1 to 6 further comprising propylene glycol or myristamidopropyl dimethylamine.

8. The composition of claims 1 to 7 further comprising hydroxypropylmethyl cellulose or hydroxylpropyl guar, and a borate or mixed borate/phosphate buffer system.

9. The composition of claims 1 to 8 wherein the disuccinate is a silver or zinc salt.

10. The composition of claims 1 to 9, wherein the composition further includes one or more active pharmaceutical agents selected from the group consisting of anti-inflammatory agents, antibiotics, immunosuppressive agents, antiviral agents, antifungal agents, anesthetics and pain killers, anticancer agents, anti-glaucoma agents, peptide and proteins, and anti-allergy agents.

11. The use of the composition of claims 1 to 9 to clean, disinfect or package contact lenses, or to preserve an ophthalmic composition.

12. An ophthalmic composition according to claims 1 to 10 for use in the treatment of people suffering from temporary or chronic eye conditions in which the eye's tear system fails to provide adequate tear volume or tear film stability.

13. The ophthalmic composition according to claim 12 for use in the treatment of patients with dry eye, wherein the ophthalmic composition is to be administered to the patient's eye, eye lid or to the skin surrounding the patient's eye.

14. Use of a composition according to any one of claims 1 to 10 for the preparation of a pharmaceutical composition for treating people suffering from temporary or chronic eye conditions in which the eye's tear system fails to provide adequate tear volume or tear film stability.

## Patentansprüche

1. Eine ophthalmische Zusammensetzung, umfassend:
ein Disuccinat der Formel I oder ein entsprechendes Salz davon: 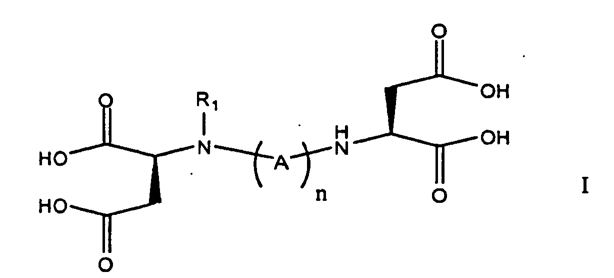 wobei R₁ ausgewählt ist aus Wasserstoff, Alkyl oder -C(O)Alkyl, wobei das Alkyl ein bis zwölf Kohlenstoffe aufweist und gegebenenfalls ein oder mehrere Sauerstoffatome aufweist,
A einen Methylenrest oder einen Oxyalkylenrest darstellt und n von 2 bis 10 ist;
einen antimikrobiellen Bestandteil; und
ein Tonizitätsmittel, wobei das Tonizitätsmittel eine Osmolalität der Zusammensetzung von 200 mOs/kg bis 420 mOsm/kg gewährleistet.

2. Die Zusammensetzung gemäß Anspruch 1, wobei das Disuccinat S,S-Ethylendiamindisuccinat ist.

3. Die Zusammensetzung gemäß Anspruch 1, wobei A Methylen darstellt und n für 2, 3 oder 4 steht und das Alkyl R₁ acht bis zwölf Kohlenstoffe aufweist.

4. Die Zusammensetzung gemäß Anspruch 1 oder 3, wobei der antimikrobielle Bestandteil ein kationischer antimikrobieller Bestandteil ist, ausgewählt aus der Gruppe bestehend aus α-[4-Tris(2-hdyroxyethyl)ammoniumchlorid-2-butenyl]poly[1-dimethylammoniumchlorid-2-butenyl]-ω-tris(2-hydroxyethyl)-ammoniumchlorid, Benzalkoniumhalogeniden, Alexidin und Salzen davon, Salzen von Chlorhexidin, Hexamethylenbiguaniden und Salzen davon und ihren Polymeren, und Gemischen davon.

5. Die Zusammensetzung gemäß den Ansprüchen 1 bis 3, wobei der antimikrobielle Bestandteil ausgewählt ist aus der Gruppe bestehend aus Poly(hexamethylenbiguanid), welches von 0,01 ppm bis 3 ppm vorhanden ist, α-[4-Tris(2-hydroxyethyl)ammoniumchlorid-2-butenyl]poly[1-dimethylammoniumchlorid-2-butenyl]-ω-tris(2-hydroxyethyl)ammoniumchlorid, welches von 1 ppm bis 10 ppm vorhanden ist, und jeglichem Gemisch davon.

6. Die Zusammensetzung gemäß den Ansprüchen 1 bis 5, ferner umfassend Dexpanthenol, Sorbit, Asparaginsäure, Glycolsäure, 2-Amino-2-methyl-1,3-propandiol oder jegliches Gemisch davon.

7. Die Zusammensetzung gemäß den Ansprüchen 1 bis 6, ferner umfassend Propylenglycol oder Myristamidopropyldimethylamin.

8. Die Zusammensetzung gemäß den Ansprüchen 1 bis 7, ferner umfassend Hydroxypropylmethylcellulose oder Hydroxylpropylguar und ein Borat oder gemischtes Borat/Phosphat-Puffersystem.

9. Die Zusammensetzung gemäß den Ansprüchen 1 bis 8, wobei das Disuccinat ein Silber- oder Zinksalz ist.

10. Die Zusammensetzung gemäß den Ansprüchen 1 bis 9, wobei die Zusammensetzung ferner ein oder mehrere pharmazeutische Wirkstoffe einschließt, ausgewählt aus der Gruppe bestehend aus entzündungshemmenden Mitteln, Antibiotika, immunsuppressiven Mitteln, antiviralen Mitteln, Antipilzmitteln, Anästhetika und Schmerzmitteln, Antikrebsmitteln, Anti-Glaukom-Mitteln, Peptid und Proteinen und Anti-Allergie-Mitteln.

11. Die Verwendung der Zusammensetzung gemäß den Ansprüchen 1 bis 9, um Kontaktlinsen zu reinigen, desinfizieren oder zu verpacken, oder um eine ophthalmische Zusammensetzung zu konservieren.

12. Eine ophthalmische Zusammensetzung gemaß den Ansprüchen 1 bis 10 zur Verwendung bei der Behandlung von Personen, die an temporären oder chronischen Augenzuständen leiden, bei denen das Tränensystem des Auges kein adäquates Tränenvolumen oder keine Tränenfilm-Stabilität bereitstellen kann.

13. Die ophthalmische Zusammensetzung gemäß Anspruch 12 zur Verwendung bei der Behandlung von Patienten mit trockenem Auge, wobei die ophthalmische Zusammensetzung in das Auge, Augenlid des Patienten oder auf die Haut, die das Auge des Patienten umgibt, verabreicht wird.

14. Verwendung einer Zusammensetzung gemäß einem der Ansprüche 1 bis 10 zur Herstellung einer pharmazeutischen Zusammensetzung zur Behandlung von Personen, die an temporären oder chronischen Augenzuständen leiden, bei denen das Tränensystem des Auges kein adäquates Tränenvolumen oder keine Tränenfilm-Stabilität bereitstellen kann.

## Revendications

1. Composition ophtalmique comprenant :
un disuccinate de formule I ou un sel correspondant de celui-ci : 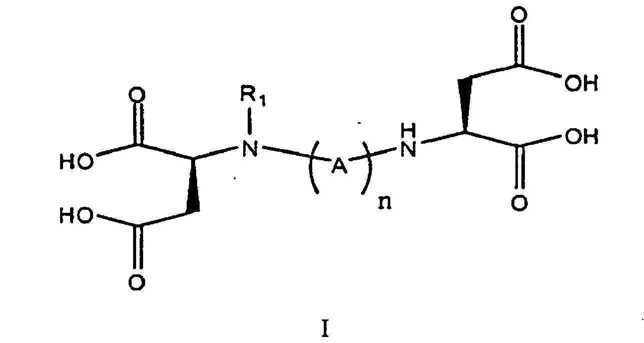 dans laquelle R₁ est choisi parmi l'hydrogène et les radicaux alkyle et -C(O)-alkyle, le fragment alkyle ayant de 1 à 12 atomes de carbone et portant éventuellement un ou plusieurs atomes d'oxygène,
A est un groupe méthylène ou un groupe oxyalkylène, et n vaut de 2 à 10;
un composant antimicrobien ; et
un agent de tonicité, lequel agent de tonicité confère une osmolalité de la composition de 200 mOs/kg à 420 mOsm/kg.

2. Composition selon la revendication 1, dans laquelle le disuccinate est le S,S-disuccinate d'éthylène-diamine.

3. Composition selon la revendication 1, dans laquelle A est le méthylène et n vaut 2, 3 ou 4, et l'alkyle R₁ a de 8 à 12 atomes de carbone.

4. Composition selon la revendication 1 ou 3, dans laquelle le composant antimicrobien est un composant antimicrobien cationique choisi dans le groupe constitué par l'α-[chlorure de 2-butényl-[4-tris(2-hydroxyéthyl)ammonium]poly[chlorure de 2-butényl-1-diméthyl-ammonium]-ω-chlorure de tris(2-hydroxyéthyl)ammonium, les halogénures de benzalkonium, l'alexidine et ses sels, les sels de chlorhexidine, les hexaméthylènebiguanides et leurs sels, et leurs polymères, ainsi que leurs mélanges.

5. Composition selon les revendications 1 à 3, dans laquelle le composant antimicrobien est choisi dans le groupe constitué par le poly(hexaméthylènebiguanide), qui est présent à raison de 0,01 ppm à 3 ppm, l'α-[chlorure de 2-butényl-[4-tris(2-hydroxyéthyl)ammonium]poly[chlorure de 2-butényl-1-diméthylammonium]-ω-chlorure de tris(2-hydroxyéthyl)ammonium, qui est présent à raison de 1 ppm à 10 ppm, et l'un quelconque de leurs mélanges.

6. Composition selon les revendications 1 à 5, comprenant en outre du dexpanthénol, du sorbitol, de l'acide aspartique, de l'acide glycolique, du 2-amino-2-méthyl-1,3-propanediol, ou l'un quelconque de leurs mélanges.

7. Composition selon les revendications 1 à 6, comprenant en outre du propylèneglycol ou de la myristamidopropyl-diméthylamine.

8. Composition selon les revendications 1 à 7, comprenant en outre de l'hydroxypropylméthylcellulose ou de l'hydroxypropyl-guar, et un système tampon borate ou borate/phosphate mixte.

9. Composition selon les revendications 1 à 8, dans laquelle le disuccinate est un sel d'argent ou de zinc.

10. Composition selon les revendications 1 à 9, laquelle composition contient en outre un ou plusieurs agents pharmaceutiques actifs choisis dans le groupe constitué par les agents anti-inflammatoires, les antibiotiques, les agents immunodépresseurs, les agents antiviraux, les agents antifongiques, les anesthésiques et antidouleurs, les agents anticancéreux, les agents anti-glaucome, les peptides et protéines, et les agents antiallergiques.

11. Utilisation de la composition des revendications 1 à 9 pour nettoyer, désinfecter ou conditionner des lentilles de contact, ou pour conserver une composition ophtalmique.

12. Composition ophtalmique selon les revendications 1 à 10, pour une utilisation dans le traitement de personnes souffrant d'états oculaires temporaires ou chroniques dans lesquels le système lacrymal de l'oeil échoue à fournir un volume adéquat de larmes ou une stabilité du film lacrymal.

13. Composition ophtalmique selon la revendication 12, pour une utilisation dans le traitement de patients ayant les yeux secs, laquelle composition ophtalmique est destinée à être administrée à l'oeil du patient, à la paupière du patient ou à la peau entourant l'oeil du patient.

14. Utilisation d'une composition selon l'une quelconque des revendications 1 à 10 pour la préparation d'une composition pharmaceutique destinée à traiter des personnes souffrant d'états oculaires temporaires ou chroniques dans lesquels le système lacrymal de l'oeil échoue à fournir un volume adéquat de larmes ou une stabilité du film lacrymal.
